# EUROPEAN PATENT SPECIFICATION

(11) **EP 2 999 778 B1**
(45) Date of publication and mention of the grant of the patent: **16.06.2021**
(21) Application number: 13859774.5
(22) Date of filing: 09.12.2013
(51) Int. Cl.: B01F 3/08, B01F 13/00, B01L 3/00, B01L 3/02

(54) **COMMON PORT EMULSION GENERATION ARTICLE, SYSTEM AND METHOD**
EMULSIONSERZEUGUNGSARTIKEL, -SYSTEM UND -VERFAHREN MIT GEMEINSAMEM PORT
ARTICLE, SYSTÈME ET PROCÉDÉ DE GÉNÉRATION D'ÉMULSION D'ORIFICE COMMUN

(30) Priority: 07.12.2012 US 201261734952 P; 11.09.2013 US 201314024145
(43) Date of publication of application: 30.03.2016
(73) Proprietor: Cypho, Inc., Long Beach, CA 90803 (US)
(72) Inventor: GRAY, Mark, A., Long Beach, CA 90803 (US)
(74) Representative: Clark, Jane Anne
(86) International application number: PCT/US2013/073957
(87) International publication number: WO 2014/089579

(56) References cited:
- WO-A1-2011/129697
- WO-A2-2005/073410
- GB-A- 2 453 585
- US-A- 5 061 741
- US-A1- 2005 202 489
- US-A1- 2011 086 780
- US-A1- 2012 190 032
- US-A1- 2012 190 032

## Description

### BACKGROUND OF THE INVENTION

1. Field of the invention. The present invention relates generally to analytical tools and methods. More particularly, the present invention relates to apparatus, systems, and methods for forming emulsion droplets suitable for use in ePCR and other analytical protocols.

Emulsions are used in a variety of biological laboratory applications. Chief among them is emulsion Polymerase Chain Reaction (ePCR), which has spurred the development of a number of commercial sample preparation platforms. There are two basic approaches to ePCR emulsion generation: agitate in a shaker or use a microfluidic system. Shaking reagents in a small disposable tube is inexpensive and precludes cross-contamination, but on the down side, the droplets generated are not uniform. Alternatively, microfluidic systems produce highly uniform droplets by passing fluids through microscopic, precision-manufactured pathways that are typically realized in planar layers of material. The major drawback of these systems is that are inherently expensive and do not scale well.

For these reasons, it would be desirable to provide alternative and improved methods, apparatus, and systems for performing ePCR in scalable and economically efficient platforms.

2. Description of the Background Art. US Patent Publication Nos. 2012/0190032 and 2011/0086780 describe systems for generation small droplet emulsions for performing ePCR and other procedures. See also US Patent No. 7,985,058 and WO 2012/142192.

US2011/0086780 discloses an article and a method comprising some features of the article according to claim 1 and the method according to claim 11. It describes a system, including method and apparatus, for forming an array of emulsions. The system may include a plate providing an array of emulsion production units each configured to produce a separate emulsion and each including a set of wells interconnected by channels that intersect to form a site of droplet generation. Each set of wells, in turn, may include at least one first input well to receive a continuous phase, a second input well to receive a dispersed phase, and an output well configured to receive from the site of droplet generation an emulsion of droplets of the dispersed phase disposed in the continuous phase.

US2012/0190032 describes a system, including method and apparatus, for generating droplets suitable for droplet-based assays. The disclosed systems may include either one-piece or multi-piece droplet generation components configured to form sample-containing droplets by merging aqueous, sample-containing fluid with a background emulsion fluid such as oil, to form an emulsion of sample-containing droplets suspended in the background fluid. In some cases, the disclosed systems may include channels or other suitable mechanisms configured to transport the sample-containing droplets to an outer region, so that subsequent assay steps may be performed.

GB2453585 describes a method of loading at least one sample into a conduit by way of a probe where steps are taken to ensure that an immiscible fluid remains between the probe and the sample. This may be achieved in any of three embodiments: i) wherein an immiscible liquid is provided covering the sample, moving the probe such that its orifice is below the level of the immiscible fluid and close to, but not touching, the surface of the sample liquid, and aspirating liquid sufficiently fast that both sample liquid and immiscible liquid are drawn into the bore of the probe; ii) covering each sample with an immiscible liquid, moving the probe so that its tip is below the surface of the sample, aspirating liquid into the probe before the probe makes contact with the sample liquid and raising the probe above the surface of the sample; iii) covering the sample with an immiscible liquid, providing a probe that possesses a main bore for aspirating the sample and at least one additional bore for ejecting the immiscible liquid, moving the top of the probe to below the level of the sample and aspirating liquid. A probe in the form of a tube possessing a flared, chamfered or radiused tube is also disclosed. The method preferably helps prevent contamination in sampling liquids for the analysis, synthesis, and crystallization etc. of samples.

### SUMMARY OF THE INVENTION

The present invention is set out in the appended claims. Described herein is a technology that combines the benefits of both prior approaches and directly addresses the needs of ePCR sample preparation. The present disclosure provides a precision microfluidic emulsion system that can be produced as an inexpensive disposable device. The preferred embodiment of the preesnt disclosure pairs precise but low-cost tubing and a simple injection-molded part to form a disposable device that is simple to use and produces highly uniform emulsions at a significantly lower cost per sample.

Described herein are methods, articles, and systems for forming emulsion droplets from an aqueous phase and an organic phase. While the aqueous phase will typically comprise nucleic acids, such as double-stranded DNA, and the organic phase will comprise an oil which together form emulsion droplets that are suitable for performing ePCR, the present disclosure will be useful for forming emulsion droplets for any analytical or other purpose. The methods, articles, and systems of the present disclosure are particularly advantageous as they simplify the procedures used for forming ePCR emulsion droplets and allow for such emulsion droplets to be formed with relatively inexpensive equipment while producing droplets of highly uniform characteristics.

In a first aspect of the present disclosure, a method for forming emulsion droplets comprises introducing an aqueous phase and an organic phase into a reservoir. The reservoir may be an open reservoir free from internal barriers or partitions, allowing the aqueous phase and organic phase to come into intimate contact and to eventually separate based on differences in their densities and other physical characteristics.
Alternatively, the reservoir may have regions defined by internal barriers, partitions, or the like, which allow each phase to be inserted within the reservoir into the same and/or different regions created by the barriers or partitions. In all cases, the reservoir will include at least one common inlet port through which both phases can be introduced at a common region within the reservoir so that automated pipetting systems need only access a single location within the reservoir.
Alternatively, in other embodiments, the two phases can be introduced into different regions within the reservoir but through the common inlet port, albeit with a possible loss of pipetting efficiency.

After the aqueous phase and the organic phase have been introduced into the reservoir, the phases will be caused to flow as separate streams from the reservoir. A variety of specific fluidic flow paths and components may be provided to effect such separation and flowing of the two phases. After the two streams flow from the reservoir, they are then combined to form the emulsion droplets which may be then be dispensed and collected, typically in a separate receptacle.

In ePCR and other DNA amplification methods, the organic phase will typically be introduced into the reservoir first followed by the aqueous phase. Flowing of the aqueous phase and the organic phase may be accomplished in a variety of ways. For example, the reservoir may be subjected to centrifuging in order to apply a force to the phases to cause them to flow through the fluidic pathways. Alternatively, the aqueous and organic phases may be caused to flow by applying a differential pressure across the reservoir. Typically, the differential pressure will be a positive pressure applied above the phases, e.g. through the common inlet port, within the reservoir. Alternatively or additionally, the differential pressure may be effected by applying a negative pressure at an outlet of the reservoir and the fluidic pathways.

Recombining the organic phase stream and the aqueous phase stream to form the emulsion droplets may also be effected in a variety of ways. Conveniently, the organic phase may be directed along an axial pathway which exits the reservoir. The aqueous phase which has separately exited the reservoir may then be directed along a lateral pathway that intersects with the axial pathway so that the phases combine to form the emulsion droplets in an efficient manner. In a specific embodiment described in detail below, the methods, the fluidic pathways are configured to achieve a coefficient of variation less than 50%, often less than 30%, and in many instances less than 10%, or better.

In a second aspect of the present disclosure, a method for forming emulsion droplets comprises introducing an aqueous phase and an organic phase into a single reservoir. By applying a force to the aqueous phase and the organic phase within the reservoir, a stream of the aqueous phase and a stream of the organic phase may be caused to flow from the reservoir. The separate aqueous phase stream and organic phase stream are then combined to form the emulsion droplets, and the emulsion droplets are dispensed into a receptacle which may be used for performing ePCR or other analyses on the emulsion droplets.

The aqueous phase and the organic phase are preferably introduced through a common inlet port on the reservoir, and the reservoir typically will have only a single common or other inlet port. The reservoir may be formed as part of a microwell plate having a plurality of individual reservoirs. Alternatively, the reservoir may be formed as a single, independent tubular or other member which consists of only a single reservoir. The methods are particularly useful for performing ePCR protocols, and the force may be applied by either centrifugation or applying a differential pressure as described above.

In specific embodiments, the organic phase is caused to flow along an axial pathway, and the aqueous phase is caused to flow along a lateral pathway, wherein the two pathways intersect to cause mixing to form the emulsion droplets. Typically, the emulsion droplets continue to flow along the axial pathway until they are dispensed into the receptacle. The droplets so formed may have a coefficient of variation less than 50%, usually less than 30%, often less than 10% or below.

After the emulsion droplets have been formed and collected in the receptacle, the droplets may be left in the receptacle and subjected to analysis. For example, the emulsion droplets may be thermocycled to perform ePCR or may be subjected to any other nucleic acid amplification procedure that can utilize such emulsions.

In a still further aspect of the present disclosure, an article for forming emulsion droplets comprises a body having an inlet port, an outlet and a reservoir configured to receive both an aqueous phase and an organic phase through the inlet port. A means for flowing an aqueous phase stream and a separate organic phase stream from the reservoir is provided, typically including a micro fluidic flow path within the body. Additionally, a means is further provided for combining the separated streams to form the emulsion droplets and dispensing them through the outlet. The means for flowing and the means for combining will typically comprise micro fluidic flow paths within the body, as described in more detail below.

According to the invention, the means for separating the aqueous phase stream and the organic phase stream comprise a hydrophobic barrier which allows the flow of the organic phase while redirecting the flow of the aqueous phase. In a specific example, the hydrophobic barrier comprises a conical element, optionally having vertical fins, which diverts the flow of droplets from the aqueous phase to a first fluidic pathway while allowing the flow of the organic phase to a second fluid pathway. Further fluidic pathways are provided in order to direct the organic phase along an axial pathway while introducing the aqueous phase into the flowing organic phase along a lateral pathway.

The articles may be formed individually in order to be removably replaced into a single receptacle. Alternatively, the articles may be formed as a group or plurality of articles in a configuration allowing their collective use, for example being formed in a micro well plate. In the latter case, the receptacle may be configured to mate with the micro well receptacle plate in order to receive and micro emulsion formed in each of the individual articles into individually isolated collection wells.

Systems according to the present disclosure may comprise any of the articles described above combined with a centrifuge configured to apply a centrifugal force to the article(s). The centrifuge will typically comprise a rotor configured to receive one or more receptacles and to apply a radial force along an axis of the receptacle when the rotor is spun.

In other embodiments, systems may comprise any of the articles described above in combination with a differential pressure generator which may be connected to the inlet port and or outlet port of the reservoir in order to apply a differential pressure to cause flow of the organic phase and the aqueous phase through the fluidic pathways of the articles.

### BRIEF DESCRIPTION OF THE DRAWINGS

Fig. 1 is an isometric, cross-sectional view of an exemplary embodiment of a single-reservoir droplet generation system.
Fig. 2 is an isometric, cross-sectional view of another embodiment of a single-reservoir droplet generation system.
Fig. 3 is a view of Fig. 2 further showing two fluids in a single reservoir.
Fig. 4 is an isometric view of various embodiments of the semi-permeable, conical barrier that separates and directs each fluid to a separate fluidic pathway.
Fig. 5 is an isometric illustration of various embodiments of the semi-permeable, conical barrier, as in Fig. 4, united with various exemplary embodiments of the reservoir.
Fig. 6 is an isometric illustration of various examples of how fluids one and two remain separate yet in intimate contact within a given reservoir, yet separated.
Fig. 7 is an isometric view of various embodiments of the segmented tube.
Fig. 8 is a cross-sectional illustration of the intersecting fluidic pathways that is the exemplary means of droplet generation for each embodiment represented in Fig. 7.
Fig. 9 is a cross-sectional view of each of the various correspondingly numbered embodiments of the segmented tube represented in Fig. 7.
Fig. 10 is an isometric, cross-sectional view of an exemplary embodiment of the article configured for centrifugation, illustrating a reservoir seated within a standard microcentrifuge tube.
Fig. 11 is an enlarged, isometric, cross-sectional view of the semi-permeable, conical barrier illustrated in Fig. 10.
Fig. 12 is an isometric view illustrating the insert and the removable, receptacle microcentrifuge tube comprising the assembly as in Fig. 10.
Fig. 13 is an exploded view of an exemplary embodiment of the article configured for centrifugation illustrating the relative positions of the inert, slotted tube, and receptacle.
Fig. 14 is a top view of an exemplary embodiment of the article as a multiwell plate.
Fig. 15 is a cross-sectional, partial view of the embodiment of the article illustrated in Fig. 14.
Fig. 16 is an isometric illustration of an exemplary embodiment of the article as in Fig. 14 seated on a typical laboratory vacuum manifold and positioned above a corresponding, receptacle multiwell plate.
Fig. 17 is an isometric, cross-sectional view of an embodiment as in Fig. 2 illustrating protective films sealing the top of the inlet and the outlet.

### DETAILED DESCRIPTION

The present disclosure comprises a system, apparatus, and methods tor generating emulsions for droplet-based assays. The advantages of the present disclosure over existing methods for generating emulsions include the ability to generate highly uniform emulsions at lower costs and with a lower propensity for errors. Various embodiments of the present disclosure are designed to be disposable units that fit within the existing footprint of high-throughput systems, such as microcentrifuge tubes and multiwell plates. The present disclosure offers a means of combining an organic phase and an aqueous phase through a single common inlet, directing each phase to a separate fluidic pathway, recombining the two phases to form emulsion droplets, and exiting the emulsion through an outlet to a receptacle that can be removed, allowing the emulsion to be further processed.

In ePCR and other DNA amplification methods, the organic phase will typically be introduced into the reservoir first followed by the aqueous phase. Flowing of the aqueous phase and the organic phase may be accomplished in a variety of ways. For example, the reservoir may be subjected to centrifuging in order to apply a force to the phases to cause them to flow through the fluidic pathways. Alternatively, the aqueous and organic phases may be caused to flow by applying a differential pressure across the reservoir. Typically, the differential pressure will be a positive pressure applied above the phases, e.g. through the common inlet port, within the reservoir. Alternatively or additionally, the differential pressure may be effected by applying a negative pressure at an outlet of the reservoir and the fluidic pathways.

Recombining the organic phase stream and the aqueous phase stream to form the emulsion droplets may also be effected in a variety of ways. Conveniently, the organic phase may be directed along an axial pathway which exits the reservoir. The aqueous phase which has separately exited the reservoir may then be directed along a lateral pathway that intersects with the axial pathway so that the phases combine to form the emulsion droplets in an efficient manner. The emulsion droplets are dispensed into a receptacle which may be used for performing ePCR or other analyses on the emulsion droplets. In a specific embodiment described in detail below, the methods, the fluidic pathways are configured to achieve a coefficient of variation less than 50%, often less than 30%, and in many instances less than 10%, or better.

The requirement to fill only one reservoir radically simplifies the protocols required to generate an emulsion via the present disclosure as compared to other existing methods. Rather than filling multiple reservoirs with various phases, the article disclosed here requires only a simple ordering of two phases, organic and then aqueous, with lower volumes required of each. The aqueous phase will typically comprise nucleic acids, such as double-stranded DNA, the organic phase will comprise an oil.

In its preferred embodiments, the article described in the present disclosure, whether incorporated into single test tubes, multiwell plates, or proprietary cartridges, can be injection-molded as part of a single unit. This results in significantly reduced manufacturing costs, thereby enabling a disposable device.

### I. DEFINITIONS

Inlet: An opening in the device, such as the open top of a container, that accepts reagents.
Reservoir: A bulk fluid retention area; the internal space of a container.
Semi-permeable barrier: A structure that directs uses their innate physiochemical properties to separate fluid one and fluid two into distinct fluidic pathways.
Slotted tube: A tubular structure or pipe with an outer diameter of not more than 1mm and an inner diameter of not more than 1 micrometer that is pierced by a lateral hole. The lateral hole runs perpendicular to the tube central axis and is dimensioned such that it fully bisects the inner diameter but not the outer diameter of the tube; the lateral hole is flanked on two sides by material that provides structural support to keep the tube segments from separating.
Lateral hole: A window shaped through-hole in a Slotted tube.
Pierced tube upper: The portion of a Slotted tube that is upstream of the Lateral hole.
Pierced tube lower: The portion of the Slotted tube that is downstream of the Lateral hole.
Tube fixture point: The point at which the outer diameter of the tube is physically attached to the device substrate.
Outlet: An opening in the device where fluids emerge, for example, as an emulsion of a first fluid and a second fluid.
Centrifuge tube: A tube designed for use with fluids in a centrifuge. As an example, microcentrifuge tubes are typically disposable, polymeric, capped tubes with an internal volume of 2 milliliters that are shaped to fit into standard centrifuge rotors.
Multiwell plate: A device comprised of a multitude of individual chambers that is broadly conforming to the specification ANSI/SLAS 2-2004 (formerly recognized as ANSI/SBS 2-2004). Interchangeable with microplate and microwell plate.
Cartridge: A polymeric device containing at least one complement of all of the elements comprising single-reservoir emulsion generation system or more slotted tubes.

### II. SYSTEM ARCHITECTURE

The present disclosure defines an article composed of 1) a single reservoir (also the inlet) to contain two fluids, an aqueous phase and an organic phase, 2) a semi-permeable barrier that enables two fluidic pathways from the single reservoir, 3) a slotted tube that recombines the two phases to create an emulsion, and 4) an outlet to a separate, removable receptacle. The article as described in this disclosure may be contained within a number of different devices, including an insert to a standard test tube, a multiwell plate, or a proprietary cartridge. This disclosure discusses the various aspects and embodiments of the article and its preferred embodiments.

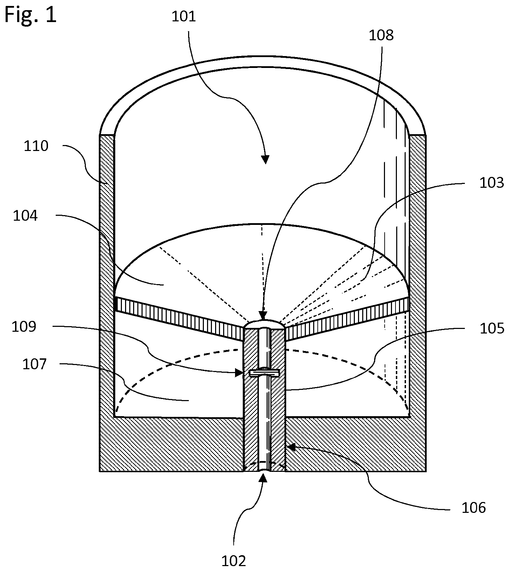

To generate an emulsion suitable for performing ePCR or other assays typically requires the combination of two fluids, one aqueous and the other organic. Existing systems generally have separate reservoirs for each fluid and microfluidic pathways that cause the two fluids to combine at some further point in the system. The present disclosure utilizes a single reservoir **110** with a single inlet **101** and a single outlet **102,** as illustrated in FIG 1. The organic phase is first added through inlet **101** to fill reservoir **110,** then the aqueous phase is added through inlet.

A semi-permeable barrier **103** comprises the lower portion of the reservoir. Its upper surface allows the organic fluid to pass through it, while retaining the aqueous fluid at its upper conical surface **104.** A slotted tube **105** is attached to the tube base **106** at the bottom **107** of the reservoir and extends upward from the outlet to the upper surface of the barrier. The inner diameter **108** of the tube is open to the upper surface of the barrier while the slot **109** is positioned such that it is only accessible to the fluid below the barrier. The end of the tube forms the outlet of the article.

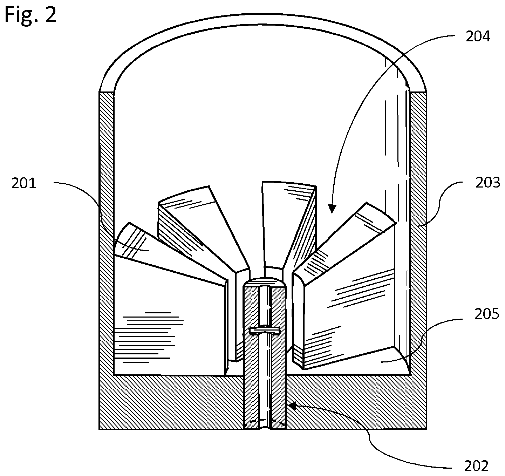

FIG **2** illustrates a preferred embodiment of the disclosure where the semi-permeable barrier **201** and slotted tube mount **202** are integral to the reservoir **203.** Advantageously, this design can be realized in a single piece using standard thermoplastic injection molding processes. The features comprising this architecture may be fully drafted, with the funnel features, reservoir structure, and tube mounting being formed from a single mold without side action. Even more advantageously, this architecture may also be produced as a collection of systems (e.g., as a multiwell plate) using a standard single-shot injection molding process.

In this preferred embodiment, an array of narrow channels **204** extend upward (in the Z axis) from the lower surface of the reservoir **205** as a closely packed collection of columnar features that together comprise a funnel-shaped upper surface.

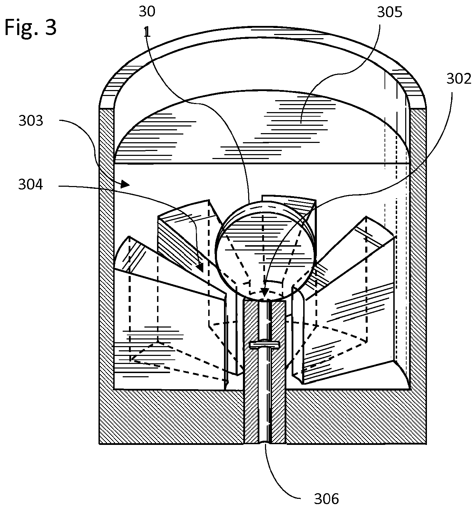

**FIG 3** illustrates the separation function provided by the semi-permeable barrier. The relative positions of the two fluids in the device are depicted. The spherical partition **301** rests at the center of the semi-permeable barrier **302** and the organic phase **303** occupies all internal spaces of the reservoir and including the internal spaces defined by the barrier, including radial channels **304** and up to the upper fluid surface **305.**

Despite the two phases comingling in a single reservoir, aqueous partitions are retained above the surface of the barrier by a combination of forces. First, gravity acts to draw the heavier aqueous fluid downward toward the barrier surface. Second, the physiochemical properties of the organic fluid in combination with the aqueous phase ensures that the aqueous reagent forms spherically shaped partitions due to surface tension where it is in contact with the organic phase. Moreover, those partitions resist breakup; larger sized partitions represent the lower energy state, and thus aqueous partitions will not tend to dissociate during operation of the device. Third, the narrow channels radiating from the center of the funnel-shaped barrier are too narrow to allow aqueous partitions to enter.

**FIG 3** illustrates how the limited width of the channels, the hydrophobic nature of the organics, and the unique geometries of the barrier ensure that each partition comprising the aqueous phase experiences the semi-permeable barrier as a contiguous, sloped surface to assure that each phase follows a separate fluidic pathway; aqueous partitions **301** displace organic fluid as they ultimately come to rest at the top of the slotted tube **302.** In this way, each fluid has access to a unique fluid pathway as they flow toward the outlet **306.**

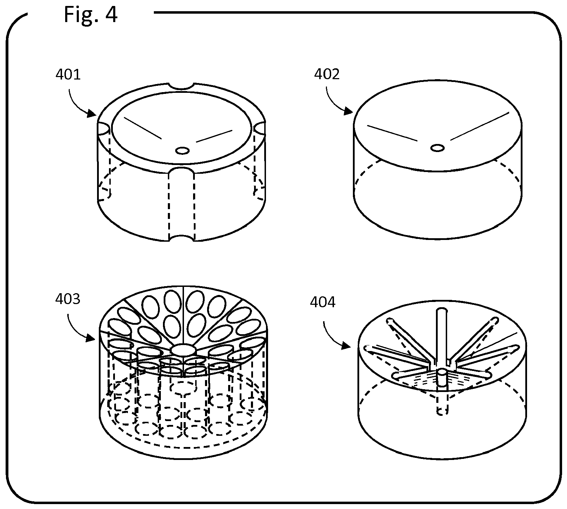

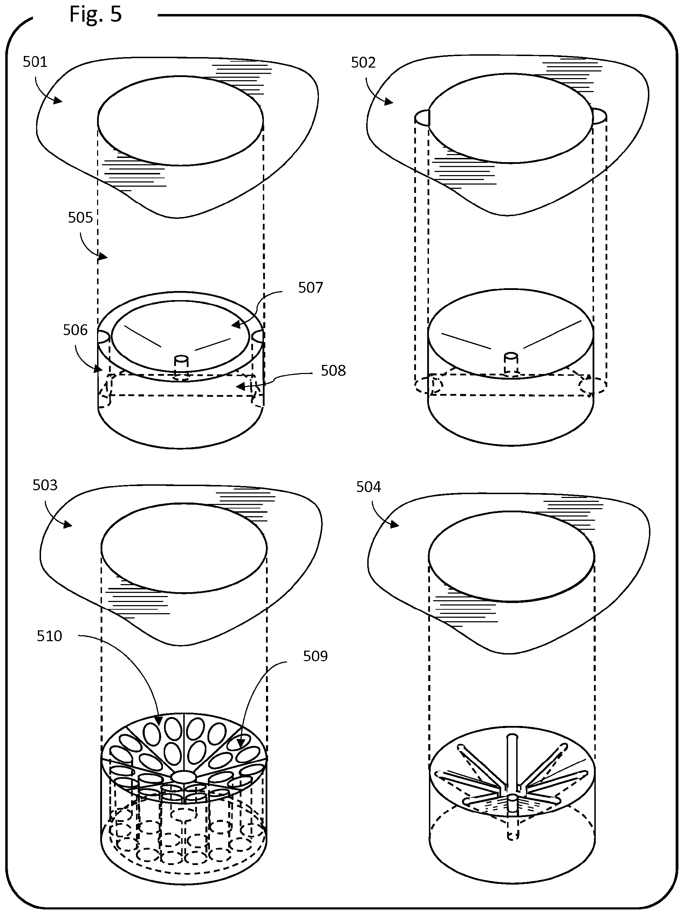

**FIG 4** illustrates an exemplary collection of selective barrier designs in accordance with the disclosure and FIG **5** shows them in situ paired with a complementary reservoir. Each of the examples provided offers a means for selectively separating the organic and aqueous reagents into two distinct flow paths. In general, organic fluid above the aqueous partition in the reservoir must pass down and around the aqueous partition. For simplicity of illustrating the point, consider that in every case there is a vertical and horizontal component to the flow, and each of the designs provided has a radial and uniform pattern with aqueous fluid directed toward an axial pathway at its center. This collection of barrier designs is not exhaustive and a multitude of useful variations in the themes outlined here are possible and are all aspects of the present disclosure.

Given that the aqueous component is retained at the center of the semi-permeable surface, variation in the design is primarily about the flow paths of the organic fluid. In examples **401** and **402,** and their corresponding reservoirs **501** and **502,** organic fluid flows down from the reservoir along vertical channels positioned at the edge of the reservoir, and then horizontally under the barrier in a lateral pathway to access the slot. In a preferred embodiment of the disclosure, example **403** and corresponding reservoir **503** illustrate a pattern of vertical shafts that provide for fluid communication between the top surface of the barrier down through the structure of the barrier to a volume that is open to the slot. In a more preferred embodiment of the disclosure, **404** and **504,** channels in the upper fluidic path allow for both the vertical and horizontal components of the flow to occur below the aqueous partition(s) without penetrating the selective barrier.

Fluids within the device are moved by pressure differentials. Motive forces can be generated by a differential gas pressure, direct force, or centrifugation. Such forces can be achieved by employing compressed gases or a vacuum pump, a piston, or a centrifuge. Some embodiments of the disclosure are more disposed to a particular device format. For example, in some instances it may be practical or convenient to incorporate the system into a cartridge. In other cases, it may be more advantageous to deploy the disclosure as a multiwell plate or centrifuge tube insert, discussed further below.

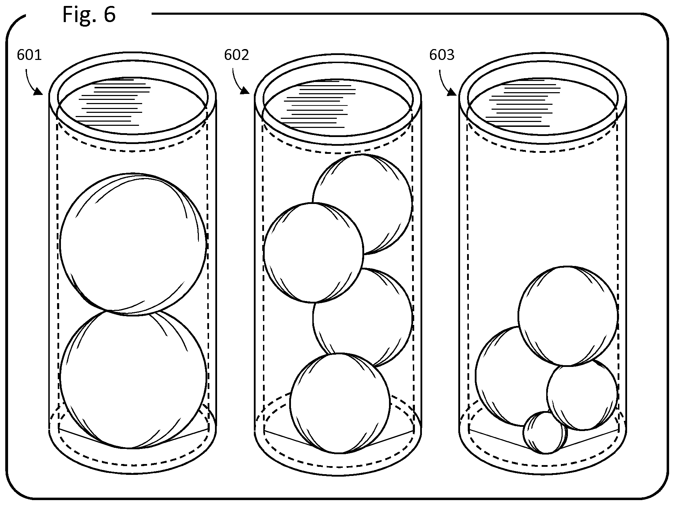

**FIG 6** illustrates the behavior of multiple aqueous partitions within the reservoir in different possible scenarios **601, 602,** and **603.** In each case, the physiochemical properties of the two fluid phases as well as the design characteristics of the reservoir and the semi-permeable barrier ensure that the aqueous partitions maintain their integrity and flow through the desired fluidic pathway, regardless of the number or shape of the aqueous partitions.

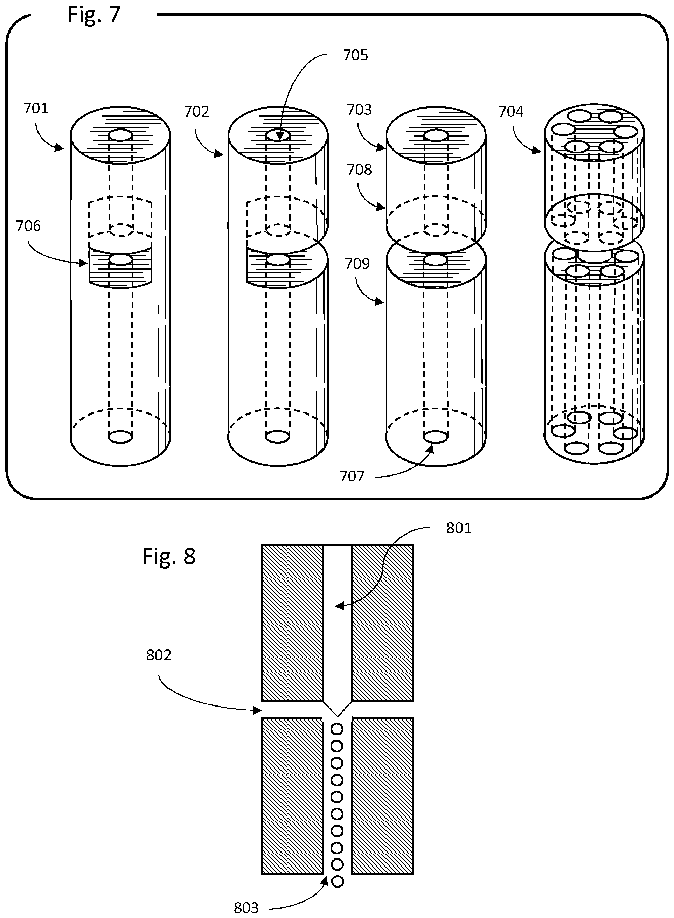

**FIG 7** illustrates various embodiments of the slotted tube**701-704,** each of which is characterized by a complete bisection of the inner diameter **705, 801** of the tube and wide access for the fluidic path from the bottom of the reservoir, through the slot or cut **706, 802,** and into the flow through the inner diameter of the tube to exit through the outlet **707**, **803,** as shown in FIG **8****.**

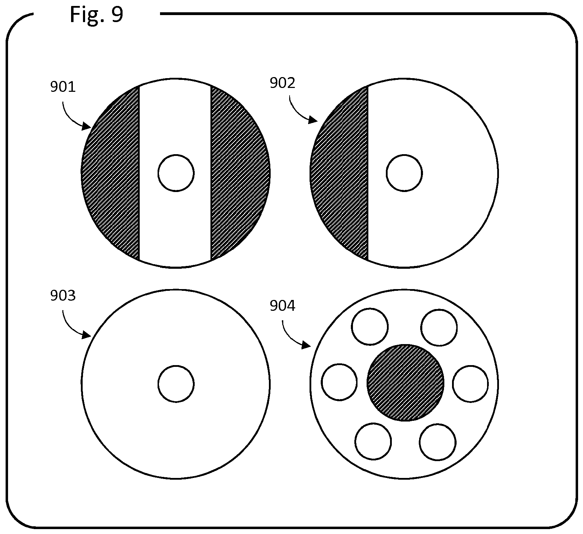

Embodiment **901** in FIG **9** shows a window through-cut, a preferred embodiment of this aspect of the present disclosure. Embodiment **902** shows a partial through-cut. Embodiment **903** shows a complete bisection, the least preferred embodiment as it requires both sections of the slotted tube to be anchored separately. Embodiment **904,** another preferred embodiment, illustrates a multi-channel tube capable of generating multiple droplets in parallel connected by a central shaft. **FIG 9** is a cross-section view of the embodiments presented in FIG **7****,** illustrating the retained material that anchors (or not) the upper **708** and lower **709** sections of the slotted tube.

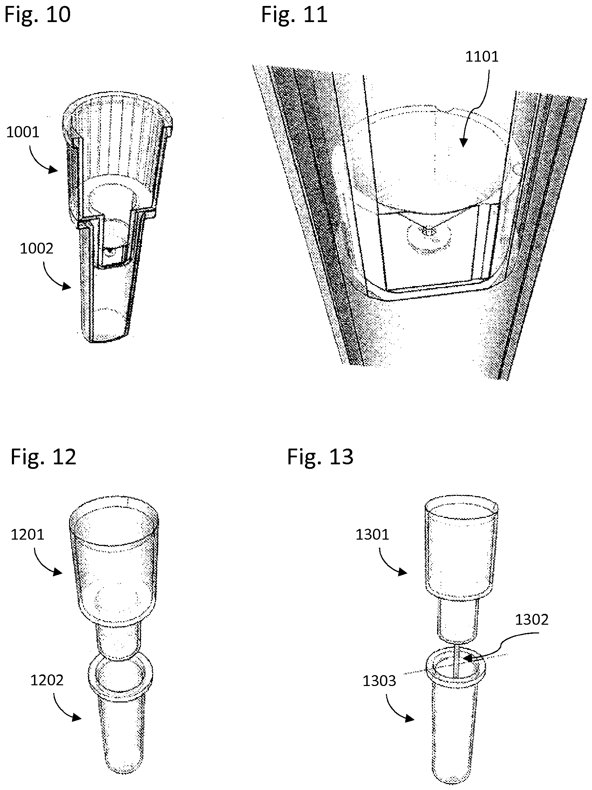

FIGS **10** through **13** illustrate a centrifugally operated device. FIG **10** depicts the device as an insert **1001** seated in a receptacle tube **1002.** FIG **11** is a more detailed view of the semi-permeable barrier **1101** with a flow pattern as described in the first example in FIG **4****.** To use the device, organic fluid is added first, followed by aqueous fluid, prior to rotation in a swinging arm or a 45-degree fixed rotor centrifuge. After centrifugation, the insert **1201** can be removed from the receptacle tube **1202,** as in FIG **12****,** so that the resultant sample can be further processed, e.g. amplified using thermocycling or another process. An exploded view comprising the reservoir **1301** and integral semi-permeable barrier structure (not shown); tube **1302** and receptacle **1303** comprising this exemplary embodiment of the disclosure are illustrated in FIG **13****.**

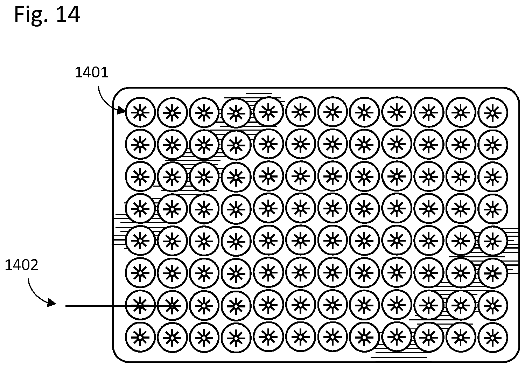

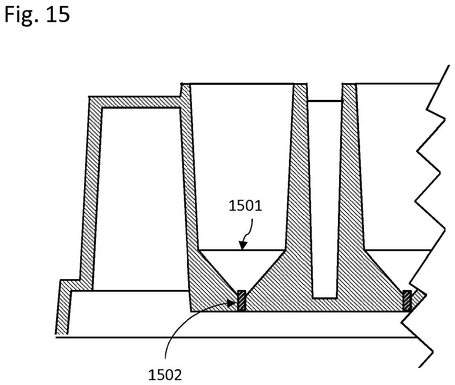

In a further embodiment, the article can be incorporated within a multiwell plate, as shown in FIG **14****.** In this embodiment, each well **1401** on the plate is a separate instance of the article. **FIG 15** provides a partial view in cross-section of a well with the semi-permeable barrier **1501** and slotted tube **1502** in situ along line **1402.**

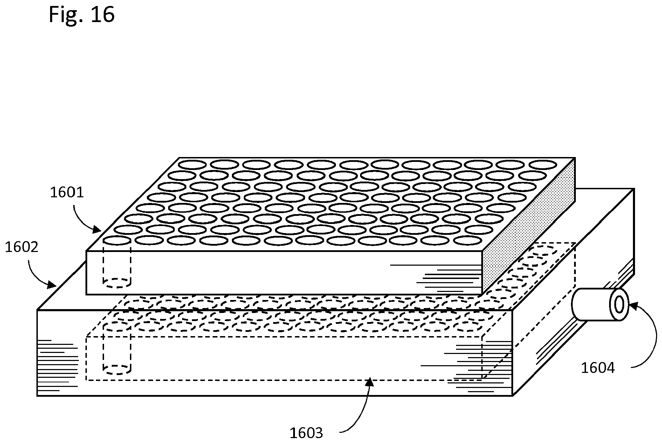

**FIG 16** further elaborates on the embodiment shown in **FIG 14****,** illustrating the embodiment in multiwell plate format **1601** with a vacuum manifold **1602** and standard multiwell plate receptacle **1603.** Application of differential pressure via the vacuum manifold via connector **1604** provides the motive force to generate the emulsion. Once the emulsion has been generated, the multiwell plate receptacle can be removed from the article described so that the resultant sample can be further processed, e.g. amplified using thermocycling or another process.

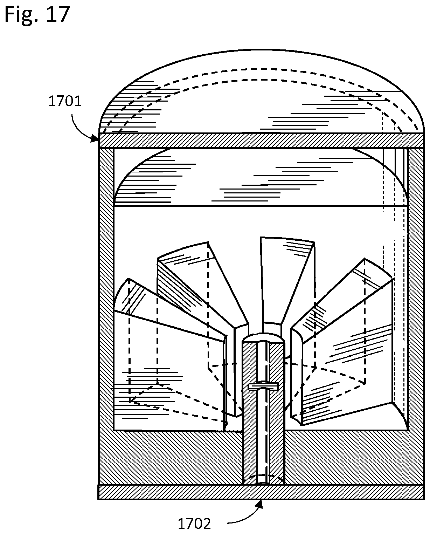

An important element of producing uniform emulsions from small fluid volumes for the purposes of ePCR or other assays is ensuring the apparatus or device used to generate the emulsion remains free from particulates or other contaminates. The present invention foresees the ability, in any embodiment, to apply a pierced or removable film over the inlet **1701,** outlet **1702,** or both, as shown in **FIG 17****.** The advantages of film applied in either or both locations are that contaminants cannot be introduced accidentally and that embodiments can be pre-loaded with a first fluid prior to use.

## Claims

1. An article for forming emulsion droplets, said article comprising:
a body having an inlet port (101), an outlet (102), and a reservoir (110) configured to receive both an aqueous phase and an organic phase through the inlet port (101);
means for flowing an aqueous phase stream and a separate organic phase stream from the reservoir, said means comprising a first fluidic pathway in the body which receives the aqueous phase stream and a second fluidic pathway which receives the organic phase stream, and wherein said means comprises a hydrophobic barrier (103) for separating the aqueous phase and the organic phase into the aqueous phase stream and the separate organic phase stream; and
means for combining (109) the streams to form emulsion droplets which are dispensed through the outlet.

2. An article as in claim 1, wherein the hydrophobic barrier comprises a conical element (104) configured to divert the flow of droplets from the aqueous phase to the first fluidic pathway while allowing the flow of the organic phase to the second fluid pathway.

3. An article as in claim 2, wherein the conical element (104) comprises a semi-permeable conical barrier.

4. An article as in claim 2, wherein the conical element (204) comprises fins which are spaced to divert flow of the aqueous phase to the first fluidic pathway while allowing flow of the organic phase to the second fluidic pathway.

5. An article as in any one of claims 1 to 4, wherein the means for combining for the streams to form emulsion droplets which are dispensed through the outlet comprises a slotted tube (105) configured to recombine the two phases to create an emulsion.

6. An article as in any one of claims 1 to 5, wherein the means for combining provides an intersection between the first fluidic pathway and the second fluidic pathway, wherein the second fluidic pathway is disposed along an axis of the body and the first fluidic pathway is disposed to deliver the aqueous phase laterally into the second fluidic pathway.

7. An article as in any one of claims 1 to 6, wherein the article is configured to be removably placed into a single receptacle.

8. A microwell plate comprising a plurality of articles as in any one of claims 1 to 7.

9. A system comprising:
an article according to any one of claims 1 to 7; and
a centrifuge comprising a rotor configured to receive a plurality of receptacles and to apply a radial force along the axes of the receptacles when the rotor is spun.

10. A system comprising:
an article according to any one of claims 1 to 7; and
a differential pressure generator having at least one port connectable to the inlet port and/or outlet of the reservoir.

11. A method for forming emulsion droplets, said method comprising:
introducing an aqueous phase and an organic phase into a reservoir (110) of a body of an article for forming emulsion droplets;
the body having an inlet port (101), an outlet (102), and a reservoir (110) configured to receive both the aqueous phase and the organic phase through the inlet port (101);
flowing an aqueous phase stream and an organic phase stream from the reservoir and using a hydrophobic barrier (103) to separate the aqueous phase stream into a first fluidic pathway of the body and the organic phase stream into a second fluidic pathway of the body; and
recombining (109) the separate organic phase stream and the aqueous phase stream to form the emulsion droplets which are dispensed through the outlet.

12. A method as in claim 11, wherein the inorganic phase is introduced first following by the aqueous phase.

13. A method as in claim 11, wherein flowing the organic phase and recombining the organic phase and the aqueous phase comprise centrifuging the reservoir.

14. A method as in claim 11, wherein flowing the organic phase and recombining the organic phase and the aqueous phase comprise applying a differential pressure across the reservoir.

15. A method as in claim 11, wherein recombining comprises directing the organic phase along an axial pathway and directing the aqueous phase along a lateral pathway that intersects the axial pathway.

## Patentansprüche

1. Artikel zur Bildung von Emulsionströpfchen, wobei der genannte Artikel Folgendes umfasst:
einen Körper mit einer Einlassöffnung (101), einem Auslass (102) und einem Reservoir (110), konfiguriert zum Aufnehmen sowohl einer wässrigen Phase als auch einer organischen Phase durch die Einlassöffnung (101);
Mittel zum Leiten eines Stroms der wässrigen Phase und eines separaten Stroms der organischen Phase aus dem Reservoir, wobei das genannte Mittel einen den Strom der wässrigen Phase aufnehmenden ersten Strömungsweg und einen den Strom der organischen Phase aufnehmenden zweiten Strömungsweg in dem Körper umfasst, und wobei das genannte Mittel eine hydrophobe Barriere (103) zum Trennen der wässrigen Phase und der organischen Phase in den Strom der wässrigen Phase und den separaten Strom der organischen Phase umfasst; und
Mittel zum Kombinieren (109) der Ströme zum Bilden von Emulsionströpfchen, die durch den Auslass abgegeben werden.

2. Artikel nach Anspruch 1, wobei die hydrophobe Barriere ein konisches Element (104) umfasst, konfiguriert zum Umleiten des Tröpfchenstroms von der wässrigen Phase zum ersten Strömungsweg, während es den Strom der organischen Phase zum zweiten Strömungsweg zulässt.

3. Artikel nach Anspruch 2, wobei das konische Element (104) eine semipermeable konische Barriere umfasst.

4. Artikel nach Anspruch 2, wobei das konische Element (204) Rippen umfasst, die so beabstandet sind, dass sie den Strom der wässrigen Phase zum ersten Strömungsweg umleiten, während sie den Strom der organischen Phase zum zweiten Strömungsweg zulassen.

5. Artikel nach einem der Ansprüche 1 bis 4, wobei das Mittel zum Kombinieren der Ströme zum Bilden von Emulsionströpfchen, die durch den Auslass abgegeben werden, ein geschlitztes Rohr (105) umfasst, das zum Rekombinieren der beiden Phasen zum Erzeugen einer Emulsion konfiguriert ist.

6. Artikel nach einem der Ansprüche 1 bis 5, wobei das Mittel zum Kombinieren einen Schnittpunkt zwischen dem ersten Strömungsweg und dem zweiten Strömungsweg bereitstellt, wobei der zweite Strömungsweg entlang einer Achse des Körpers angeordnet ist und der erste Strömungsweg zum Zuführen der wässrigen Phase lateral in den zweiten Strömungsweg angeordnet ist.

7. Artikel nach einem der Ansprüche 1 bis 6, wobei der Artikel zum entfernbaren Platzieren in einem einzigen Gefäß konfiguriert ist.

8. Mikrotiterplatte mit mehreren Artikeln nach einem der Ansprüche 1 bis 7.

9. System, das Folgendes umfasst:
einen Artikel nach einem der Ansprüche 1 bis 7; und
eine Zentrifuge mit einem Rotor, konfiguriert zum Aufnehmen mehrerer Behälter und zum Aufbringen einer Radialkraft entlang der Achsen der Behälter, wenn der Rotor gedreht wird.

10. System, das Folgendes umfasst:
einen Artikel nach einem der Ansprüche 1 bis 7; und
einen Differenzdruckgenerator mit mindestens einer Öffnung, die mit der Einlassöffnung und/oder dem Auslass des Behälters verbunden werden kann.

11. Verfahren zum Bilden von Emulsionströpfchen, wobei das genannte Verfahren Folgendes beinhaltet:
Einleiten einer wässrigen Phase und einer organischen Phase in ein Reservoir (110) eines Körpers eines Artikels zum Bilden von Emulsionströpfchen;
wobei der Körper eine Einlassöffnung (101), einen Auslass (102) und ein Reservoir (110) aufweist, konfiguriert zum Aufnehmen sowohl der wässrigen Phase als auch der organischen Phase durch die Einlassöffnung (101);
Leiten eines Stroms der wässrigen Phase und eines Stroms der organischen Phase aus dem Reservoir und Verwenden einer hydrophoben Barriere (103) zum Trennen des Stroms der wässrigen Phase in einen ersten Strömungsweg des Körpers und den Strom der organischen Phase in einen zweiten Strömungsweg des Körpers; und
Rekombinieren (109) des separaten Stroms der organischen Phase und des Stroms der wässrigen Phase zum Bilden der Emulsionströpfchen, die durch den Auslass abgegeben werden.

12. Verfahren nach Anspruch 11, wobei zuerst die anorganische Phase, gefolgt von der wässrigen Phase eingeleitet wird.

13. Verfahren nach Anspruch 11, wobei das Leiten der organischen Phase und das Rekombinieren der organischen Phase und der wässrigen Phase das Zentrifugieren des Reservoirs beinhalten.

14. Verfahren nach Anspruch 11, wobei das Leiten der organischen Phase und das Rekombinieren der organischen Phase und der wässrigen Phase das Beaufschlagen des Reservoirs mit einem Differenzdruck beinhalten.

15. Verfahren nach Anspruch 11, wobei das Rekombinieren das Richten der organischen Phase entlang eines axialen Weges und das Richten der wässrigen Phase entlang eines den axialen Weg schneidenden lateralen Weges beinhaltet.

## Revendications

1. Article conçu pour former des gouttelettes d'émulsion, ledit article comprenant :
un corps comportant un orifice d'entrée (101), une sortie (102) et un réservoir (110) configuré pour recevoir à la fois une phase aqueuse et une phase organique via l'orifice d'entrée (101) ;
un moyen d'écoulement d'un courant de phase aqueuse et d'un courant de phase organique distinct à partir du réservoir, ledit moyen comprenant une première voie fluidique dans le corps qui reçoit le courant de phase aqueuse et une deuxième voie fluidique qui reçoit le courant de phase organique, et ledit moyen comprenant une barrière hydrophobe (103) pour séparer la phase aqueuse et la phase organique afin de produire le courant de phase aqueuse et le courant de phase organique distinct ; et
un moyen de combinaison (109) des courants pour former des gouttelettes d'émulsion qui sont distribuées via la sortie.

2. Article selon la revendication 1, dans lequel la barrière hydrophobe comprend un élément conique (104) configuré pour détourner l'écoulement des gouttelettes de la phase aqueuse vers la première voie fluidique tout en permettant l'écoulement de la phase organique vers la deuxième voie fluidique.

3. Article selon la revendication 2, dans lequel l'élément conique (104) comprend une barrière conique semi-perméable.

4. Article selon la revendication 2, dans lequel l'élément conique (204) comprend des ailettes qui sont espacées pour détourner l'écoulement de la phase aqueuse vers la première voie fluidique tout en permettant l'écoulement de la phase organique vers la deuxième voie fluidique.

5. Article selon l'une quelconque des revendications 1 à 4, dans lequel le moyen de combinaison des courants pour former des gouttelettes d'émulsion qui sont distribuées via la sortie comprend un tube fendu (105) configuré pour recombiner les deux phases afin de créer une émulsion.

6. Article selon l'une quelconque des revendications 1 à 5, dans lequel le moyen de combinaison produit une intersection entre la première voie fluidique et la deuxième voie fluidique, la deuxième voie fluidique étant disposée le long d'un axe du corps et la première voie fluidique étant disposée pour introduire la phase aqueuse latéralement dans la deuxième voie fluidique.

7. Article selon l'une quelconque des revendications 1 à 6, l'article étant configuré pour être placé de façon amovible dans un réceptacle individuel.

8. Microplaque comprenant une pluralité d'articles selon l'une quelconque des revendications 1 à 7.

9. Système comprenant :
un article selon l'une quelconque des revendications 1 à 7 ; et
une centrifugeuse comprenant un rotor configuré pour recevoir une pluralité de réceptacles et pour appliquer une force radiale le long des axes des réceptacles lorsqu'on fait tourner le rotor.

10. Système comprenant :
un article selon l'une quelconque des revendications 1 à 7 ; et
un générateur de pression différentielle comportant au moins un orifice qui peut être raccordé à l'orifice d'entrée et/ou à la sortie du réservoir.

11. Procédé de formation de gouttelettes d'émulsion, ledit procédé comprenant :
l'introduction d'une phase aqueuse et d'une phase organique dans un réservoir (110) d'un corps d'un article pour former des gouttelettes d'émulsion ;
le corps comportant un orifice d'entrée (101), une sortie (102) et un réservoir (110) configuré pour recevoir à la fois la phase aqueuse et la phase organique via l'orifice d'entrée (101) ;
l'écoulement d'un courant de phase aqueuse et d'un courant de phase organique à partir du réservoir et l'utilisation d'une barrière hydrophobe (103) pour séparer le courant de phase aqueuse en l'introduisant dans une première voie fluidique du corps et le courant de phase organique en l'introduisant dans une deuxième voie fluidique du corps ; et
la recombinaison (109) du courant de phase organique distinct et du courant de phase aqueuse pour former les gouttelettes d'émulsion qui sont distribuées via la sortie.

12. Procédé selon la revendication 11, dans lequel la phase inorganique est introduite en premier, suivie de la phase aqueuse.

13. Procédé selon la revendication 11, dans lequel l'écoulement de la phase organique et la recombinaison de la phase organique et de la phase aqueuse comprennent une centrifugation du réservoir.

14. Procédé selon la revendication 11, dans lequel l'écoulement de la phase organique et la recombinaison de la phase organique et de la phase aqueuse comprennent l'application d'une pression différentielle à travers le réservoir.

15. Procédé selon la revendication 11, dans lequel la recombinaison comprend le fait de diriger la phase organique le long d'une voie axiale et de diriger la phase aqueuse le long d'une voie latérale qui croise la voie axiale.
